(19) Europäisches Patentamt
European Patent Office
Office européen des brevets

(11) Publication number: 0 004 733 A2

(12) **EUROPEAN PATENT APPLICATION**

(21) Application number: 79300460.7

(22) Date of filing: 22.03.79

(51) Int. Cl.[2]: C 07 D 231/06
A 01 N 9/22, C 07 C 49/80
//C07C49/76

(30) Priority: 31.03.78 GB 1266378

(43) Date of publication of application: 17.10.79 Bulletin 79/21

(84) Designated Contracting States: BE CH DE FR GB IT NL

(71) Applicant: IMPERIAL CHEMICAL INDUSTRIES LIMITED
Imperial Chemical House Millbank
London SW1P 3JF(GB)

(72) Inventor: Hunt, John Desmond
24 Goodwood Close
Midhurst Sussex(GB)

(74) Representative: Bishop, Nigel Douglas et al,
Imperial Chemical Industries Limited Legal Department: Patents Thames House North Millbank
London SW1P 4QG(GB)

(54) **Diaryl substituted pyrazoline carboxanilides, processes for preparing them, insecticidal compositions and uses thereof, and intermediates therefor.**

(57) Compounds of formula I where $R^1$ and $R^2$ are H, halogen, $CF_3$; $R^3$ is alkyl ($C_1$ - $C_6$), phenyl; $R^4$ is H, alkyl ($C_1$ - $C_6$); and compositions comprising them useful as insecticides.

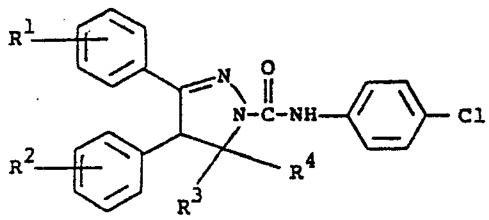

EP 0 004 733 A2

Croydon Printing Company Ltd.

0004733

30131|EP.



# DIARYL SUBSTITUTED PYRAZOLINE CARBOXANILIDES, PROCESSES FOR PREPARING THEM, INSECTICIDAL COMPOSITIONS AND USES THEREOF, AND INTERMEDIATES THEREFOR

This invention relates to pyrazoline derivatives, to methods for their preparation, to compositions comprising them and to methods of combating pests, especially insect pests therewith.

Netherlands patent application 7409433 describes 1-arylcarbamoyl-3,4-diaryl-2-pyrazolines useful as insecticides. We have found that the 5-alkyl or 5-phenyl analogues of these compounds possess superior insecticidal properties.

Accordingly, the present invention provides compounds of formula:

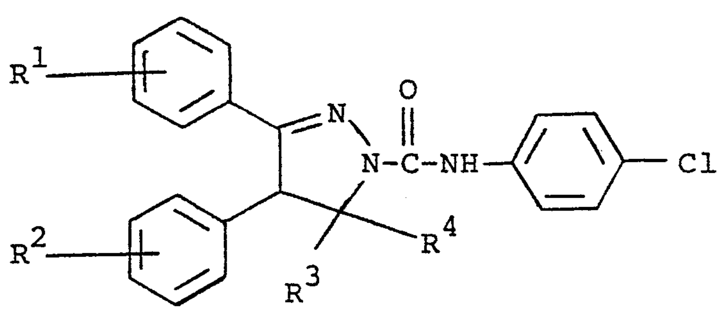

wherein $R^1$ and $R^2$ are each selected from hydrogen, halogen, and trifluoromethyl, $R^3$ is alkyl of up to six carbon atoms or phenyl and $R^4$ is hydrogen or alkyl of up to six carbon atoms.

Preferably either $R^1$ and $R^2$ are both 4-chloro or one is 4-chloro and the other is 3-trifluoromethyl, $R^3$ is preferably methyl, ethyl, propyl or phenyl, and $R^4$ is preferably hydrogen or methyl.

Examples of specific compounds according to the invention, all of which conform to formula I, are set out in Table I below. The Table gives the meaning for $R^1$, $R^2$, $R^3$ and $R^4$ for each compound, together with its melting point (°C) and an indication of the stereochemistry where this has been determined.

TABLE I

| Compound No. | $R^1$ | $R^2$ | $R^3$ | $R^4$ | Melting Point (°C) |
|---|---|---|---|---|---|
| 1 | H | H | $C_6H_5$ | H | 194-195°C |
| 2 | 4-Cl | 4-Cl | $CH_3$ | H | 162-163°C (50% cis) |
| 3 | 4-Cl | 4-Cl | $CH_3$ | $CH_3$ | 175-177°C |
| 4 | $3-CF_3$ | 4-Cl | $CH_3$ | H | 164-165°C (100% cis) |
| 5 | 4-Cl | 4-Cl | $C_2H_5$ | H | 215-218°C |
| 6 | $3-CF_3$ | 4-Cl | $CH_3$ | $CH_3$ | 83-86°C |
| 7 | $4-CF_3$ | 4-Cl | $CH_3$ | H | 148-150°C (89% trans) |
| 8 | $4-CF_3$ | 4-Cl | $CH_3$ | H | 141-143°C (43% cis) |
| 9 | 4-Cl | 4-Cl | $n-C_3H_7$ | H | 204°C |
| 10 | 4-Cl | 4-Cl | $C_6H_5$ | H | 245°C |
| 11 | $3-CF_3$ | 4-Cl | $CH_3$ | H | 98-100°C (100% trans) |

The compounds of the invention wherein $R^3$ and $R^4$ are not identical are capable of existing in different geometrically isomeric forms, and each geometrically

isomeric form is capable of existing as a pair of diastereoisomers. In addition since the carbon atom at the 4-position in the pyrazoline ring is asymmetrically substituted it also constitutes a chiral centre giving rise to optical isomers. The scope of the present invention is to be construed as extending to include all possible isomers of the compounds of formula I either in an isolated state substantially free from other isomers or as mixtures of isomers including racemic mixtures.

The compounds of the invention may be prepared by the reaction of a compound of formula:

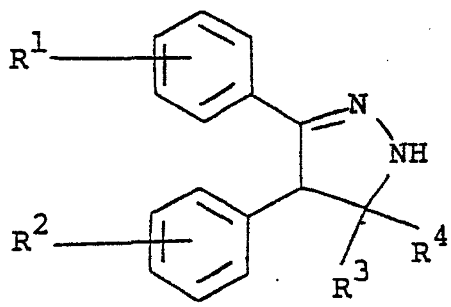

(II)

where $R^1$, $R^2$, $R^3$ and $R^4$ have any of the meanings given hereinabove with 4-chlorophenyl isocyanate. This process is conveniently carried out using a solvent or diluent which is inert with respect to the reactants. Suitable solvents or diluents include aromatic hydrocarbons such as toluene or xylene, aliphatic esters such as diethyl ether, tetrahydrofuran or dioxan, or polar aprotic diluents such as dimethylformamide or dimethylsulphoxide. The reaction may be catalysed by small quantities of suitable aprotic bases, for example N-methylmorpholine, pyridine or triethylamine. The reaction normally proceeds well at or below the ambient temperature (15 to 25°C), but if desired the reactant mixture can be heated to a temperature within the range 40 to 70°C to accelerate or complete the reaction.

The products of formula I are normally well defined crystalline solids at the ambient temperature, and may be purified by recrystallisation from suitable solvents. Where *cis* and *trans* isomers are present these may be

separated by virtue of their differential solubility characteristics by fractional crystallisation or by chromatographic techniques.

The compounds of formula II used as intermediates for the preparation of the compounds of formula I are also novel compounds and may be prepared by reacting a compound of formula:

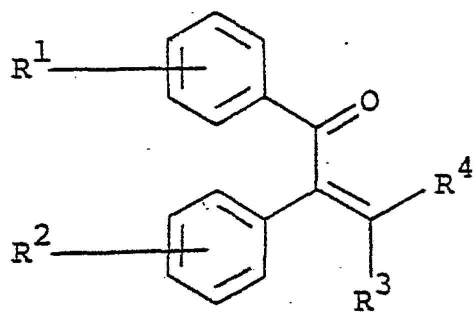

(III)

with hydrazine (optionally in the form of an acid addition salt or hydrate thereof). The reaction is conveniently conducted in the presence of a solvent such as a saturated lower aliphatic alcohol containing up to five carbon atoms (for example, methanol, ethanol, or *t*-butanol). The reaction is preferably conducted in the absence of light and under an oxygen-free inert atmosphere, for example under a nitrogen atmosphere, and at the ambient temperature.

The compounds of formula III may be made by a three step procedure from the corresponding deoxybenzoin. In the first step a deoxybenzoin of formula:

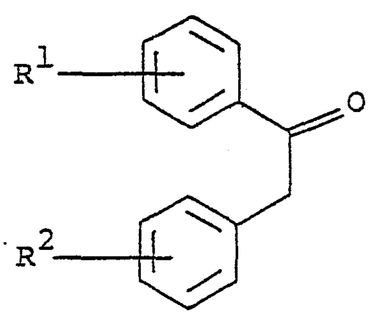

(IV)

is subjected to alkylation with a compound of formula:

$$R^3R^4CHX$$

where $R^1$, $R^2$, $R^3$ and $R^4$ are as defined hereinabove and X

is a suitable leaving group, for example a halo (preferably chloro, bromo or iodo) radical or tosyl group, the reaction preferably being conducted in the presence of a base, for example an alkali metal alkoxide, and a solvent, for example the corresponding alcohol to the alkoxide. The product of the first step is a compound of formula:

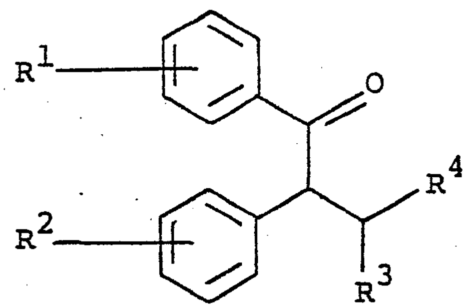

(V)

which in the second step is halogenated at the carbon atom bearing $R^3$ and $R^4$. This is conveniently achieved by, for example, bromination in carbon tetrachloride, leading to a compound of formula:

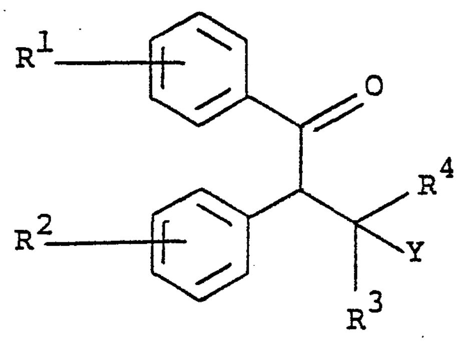

(VI)

where Y is a halo radical, which in the third step of the process is subjected to dehydrohalogenation to give the compound of formula III. Although this might be achieved by base treatment, for example treatment with tertiary amines or pyridine, a particularly convenient reagent for effecting dehydrohalogenation in these compounds is lithium chloride in dimethylformamide.

An alternative process for the preparation of the compounds of formula III, particularly those where one of $R^3$ and $R^4$ is a phenyl or substituted phenyl grouping consists of condensing a deoxybenzoin of formula IV with a compound of formula:

0004733

$R^3R^4C=O$

The deoxybenzoins used as starting materials for the above process may be prepared by any of the known procedures for the preparation of deoxybenzoins, such as for example those set out in J. Med Chem 14, 1138 (1971), J. Chem. Soc. 1123 (1935), ibid, 577 (1926), or Helv. Chem. Acta, 21, 1084 (1938).

The compounds of formula I may be used to combat and control infestations of insect pests and also other invertebrate pests, for example, acarine pests. The insect and acarine pests which may be combated and controlled by the use of the invention compounds include those pests associated with agriculture (which term includes the growing of crops for food and fibre products, horticulture and animal husbandry), forestry, the storage of products of vegetable origin, such as fruit, grain and timber, and also those pests associated with the transmission of diseases of man and animals.

In order to apply the compounds to the locus of the pests they are usually formulated into compositions which include in addition to the insecticidally active ingredient or ingredients of formula I suitable inert diluent or carrier materials, and/or surface active agents. The compositions may also comprise another pesticidal material, for example another insecticide or acaricide, or a fungicide, or may also comprise a insecticide synergist, such as for example dodecyl imidazole, safroxan, or piperonyl butoxide.

The compositions may be in the form of dusting powders wherein the active ingredient is mixed with a solid diluent or carrier, for example kaolin, bentonite, kieselguhr, or talc, or they may be in the form of granules, wherein the active ingredient is absorbed in a porous granular material for example pumice.

Alternatively the compositions may be in the form of liquid preparations to be used as dips or sprays, which

are generally aqueous dispersions or emulsions of the active ingredient in the presence of one or more known wetting agents, dispersing agents or emulsifying agents (surface active agents).

Wetting agents, dispersing agents and emulsifying agents may be of the cationic, anionic or non-ionic type. Suitable agents of the cationic type include, for example, quaternary ammonium compounds, for example, cetyltrimethyl ammonium bromide. Suitable agents of the anionic type include, for example, soaps, salts of aliphatic monoesters or sulphuric acid, for example sodium lauryl sulphate, salts of sulphonated aromatic compounds, for example sodium dodecylbenzenesulphonate, sodium, calcium or ammonium lignosulphonate, butylnaphthalene sulphonate, and a mixture of the sodium salts of diisopropyl- and triisopropylnaphthalene sulphonates. Suitable agents of the non-ionic type include for example, the condensation products of ethylene oxide with fatty alcohols such as oleyl alcohol or cetyl alcohol, or with alkyl phenols such as octyl phenol, nonyl phenol and octyl cresol. Other non-ionic agents are the partial esters derived from long chain fatty acids and hexitol anhydrides, the condensation products of the said partial esters with ethylene oxide, and the lecithins.

The compositions may be prepared by dissolving the active ingredient in a suitable solvent, for example, a ketonic solvent such as diacetone alcohol, or an aromatic solvent such as trimethylbenzene and adding the mixture so obtained to water which may contain one or more known wetting, dispersing or emulsifying agents. Other suitable organic solvents are dimethyl formamide, ethylene dichloride, isopropyl alcohol, propylene glycol and other glycols, diacetone alcohol, toluene, kerosene, white oil, methylnaphthalene, xylenes and trichloroethylene, N-methyl-2-pyrrolidone and tetrahydro furfuryl alcohol (THFA).

The compositions to be used as sprays may also be in

the form of aerosols wherein the formulation is held in a container under pressure in the presence of a propellant such as fluorotrichloromethane or dichlorodifluoromethane. The compositions which are to be used in the form of aqueous dispersions or emulsions are generally supplied in the form of a concentrate containing a high proportion of the active ingredient or ingredients, the said concentrate to be diluted with water before use. These concentrates are often required to withstand storage for prolonged periods and after such storage, to be capable of dilution with water to form aqueous preparations which remain homogeneous for a sufficient time to enable them to be applied by conventional spray equipment. The concentrates may contain 10-85% by weight of the active ingredient or ingredients. When diluted to form aqueous preparations, such preparations may contain varying amounts of the active ingredient depending upon the purpose for which they are to be used.

For agricultural or horticultural purposes, an aqueous preparation containing between 0.0001% and 0.1% by weight of the active ingredient is particularly useful.

In use the compositions are applied to the pests, to the locus of the pests, to the habitat of the pests, or to growing plants liable to infestation by the pests, by any of the known means of applying pesticidal compositions, for example, by dusting or spraying.

The compositions of the invention are very toxic to wide varieties of insect and other invertebrate pests, including, for example, the following:-

<u>Aphis fabae</u> (aphids)
<u>Megoura viceae</u> (aphids)
<u>Aedes aegypti</u> (mosquitoes)
<u>Dysdercus fasciatus</u> (capsids)
<u>Musca domestica</u> (houseflies)
<u>Pieris brassicae</u> (white butterfly, larvae)
<u>Plutella maculipennis</u> (diamond back moth, larvae)

Phaedon cochleariae (mustard beetle)
Telarius cinnabarinus (carmine spider mite)
Aonidiella spp. (scale insects)
Trialeuroides spp. (white flies)
Blattella germanica (cockroaches)
Spodoptera littoralis (cotton leaf worm)
Chortiocetes terminifera (locusts)

The various aspects of the invention are illustrated by the following Examples:

## EXAMPLE 1

This Example illustrates the preparation of 4'-chloro-3-trifluormethyldeoxybenzoin. m-Trifluoromethyldeoxybenzonitrile (41.0g. 0.24 mole) in 150 ml dry ether was added dropwise to a Grignard reagent prepared from 48.3g (0.30 mole) of p-chlorobenzyl chloride and 7.8g (0.32 mole) of magnesium in 150 ml of dry ether. The reaction mixture was stirred, first at room temperature and then on a steam bath for 3 hours and decomposed with ice and hydrochloric acid. The product was extracted with ether, washed, dried, the solvent removed and the residue distilled to yield 4'-chloro-3-trifluoromethyldeoxybenzoin, b.p. 138-142$^{o}$C/0.45 mm Hg.

## EXAMPLE 2

This Example illustrates the preparation of 1,2,3-triphenylprop-2-ene-1-one.

A mixture of deoxybenzoin (8.4g, 0.05 mole), benzaldehyde (6.15g, 0.058 mole) and morpholine (0.4 ml) in 100 ml of dry toluene was refluxed under nitrogen for 14 hours, removing the water formed through a Dean and Stark separator. The solvent was removed under vacuum. The residue was decolourised and recrystallised from diethyl ether - 40-60 petroleum ether, to give 1,2,3-

triphenylprop-2-ene-1-one, pale yellow solid, m.p. 102°C.

EXAMPLE 3

This Example illustrates the preparation of 2-(4-chlorophenyl)-1-(3-trifluoromethylphenyl) butan-1-one.

Potassium (2.45g, 0.0625 mole) was dissolved in 100 ml of dry t-butanol under a nitrogen atmosphere, and 4'-chloro-3-trifluoromethyldeoxybenzoin (15.41g. 0.0516 mole) added and the mixture brought to reflux. After 5 minutes iodoethane (12.79g, 0.082 mole) was added and the reaction mixture boiled under reflux for 4 hours. The solvent was removed and the residual paste extracted with chloroform. The chloroform solution was evaporated leaving a residue of 2-(4-chlorophenyl)-1-(3-trifluoromethylphenyl) butan-1-one, as an oil.

EXAMPLE 4

This Example illustrates the preparation of 2-(4-chlorophenyl)-1-(3-trifluoromethylphenyl) but-2-ene-1-one.

2-(4-chlorophenyl)-1-(3-trifluoromethylphenyl) butan-1-one (16.55g, 0.051 mole) and bromine (2.7 ml, 0.051 mole) were stirred together in 100 ml of carbon tetrachloride for 40 hours at room temperature. Removal of the solvent left a red oil which was dissolved in 100 ml of dimethylformamide, lithium chloride added (8.65g, 0.204 mole) and the reaction mixture heated, with stirring, at 130°C for 3 hours. The reaction mixture was poured into water, extracted with ether; the ether extracts were washed, dried and, after the removal of the solvent, the residual oil was distilled to give 2-(4-chlorophenyl)-1-(3-trifluoromethylphenyl) butan-2-ene-1-one, b.p. 130-131°C/0.15 mm Hg.

EXAMPLE 5

This Example illustrates the preparation of cis- and trans-1-(4-chlorophenylcarbamoyl)-4-(4-chlorophenyl)-5-methyl-3-(3-trifluoromethylphenyl)pyrazoline.

2-(4-chlorophenyl)-1-(3-trifluoromethylphenyl)-but-2-ene-1-one (11.0g, 0.034 mole), hydrazine hydrate (17 ml, 0.34 mole) and methanol (200 ml) were stirred together at room temperature under a nitrogen atmosphere in a flask covered in aluminium foil to keep out the light. After 24 hours the solvent was removed and the hydrazine solution extracted with diethyl ether. The ether extracts were washed with brine, dried, filtered and p-chlorophenylisocyanate (5.22g, 0.034 mole) added. The reaction mixture was stirred at room temperature for 24 hours then filtered to remove the impurity of N,N'-bis(4-chlorophenyl)urea. The filtrate was evaporated leaving an oil which contained both cis- and trans1-(4-chlorophenylcarbamoyl)-4-(4-chlorophenyl)-5-methyl-3-(3-trifluoromethylphenyl)pyrazoline. Purification of the oil by column chromatography and recrystallisation gave the pure cis-isomer, m.p. 164-165°C and pure trans-isomer, m.p. 98-100°C.

EXAMPLE 6

By use of similar procedures to those illustrated in preceding Examples the other compounds listed in Table I were prepared from the appropriate starting materials.

EXAMPLE 7

The insecticidal activity of the compounds was demonstrated in the following test.

An aqueous emulsion containing 1000 parts per million

of the compound under test and 100 parts per million of a wetting agent ("Lissapol" NX - "Lissapol" is a registered trade mark) was sprayed on to the pests and the medium upon which they were supported using a Potter tower. The mortality of the pests was observed after 24 hours and the results are given in the following Table as a grading of from 0-3 where

0 represents less than 30% observed mortality

1 represents 30-49% observed mortality

2 represents 50-90% observed mortality

and 3 represents 90-100% observed mortality.

TABLE

| Pest Species | Compound no. (Table 1) 2 | 3 | 4 | 7 | 8 |
|---|---|---|---|---|---|
| *Musca domestica* (houseflies | 3 | 0 | 3 | 3 | 3 |
| *Spodoptera littoralis* (cotton leaf worms) | 3 | 3 | 3 | 3 | 3 |
| *Plutella xylostella* (diamond back moth, larvae) | 3 | 3 | 3 | 2 | 2 |
| *Phaedon cochleariae* (mustard beetles) | 3 | 0 | 3 | 2 | 3 |
| *Sitophilus granaria* (grain weevils) | 3 | 0 | 3 | 2 | 2 |

1. A compound of the formula:

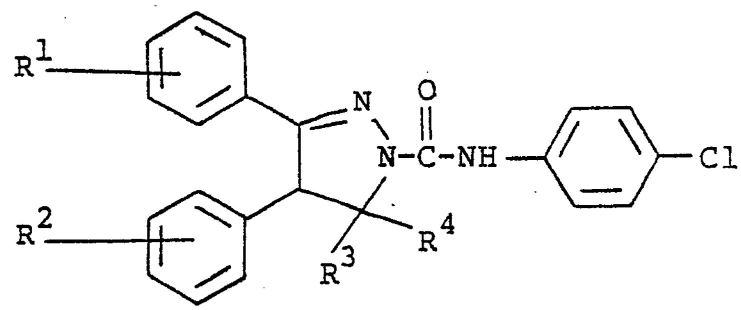

wherein $R^1$ and $R^2$ are each selected from hydrogen, halogen, and trifluoromethyl, $R^3$ is alkyl of up to six carbon atoms or phenyl and $R^4$ is hydrogen or alkyl of up to six carbon atoms.

2. A compound according to claim 1 wherein $R^1$ and $R^2$ are both 4-chloro or one is 4-chloro and the other is 3-trifluoromethyl, $R^3$ is methyl, ethyl, propyl or phenyl, and $R^4$ is hydrogen or methyl.

3. 1-(4-chlorophenylcarbamoyl)-4-(4-chlorophenyl)-5-methyl-3-(3-trifluoromethylphenyl)pyrazoline.

4. The compound of claim 3 in the form of its <u>cis</u> or <u>trans</u> isomer and substantially free from contamination by the other isomer.

5. An insecticidal composition comprising an insecticidally effective amount of a compound according to claim 1 in association with an inert diluent or carrier material.

6. A method of combating insect pests in which an insecticidally effective amount of a composition according to claim 5 is applied to the pests, the

locus of the pests, to the habitat of the pests or to growing plants liable to infestation by the pests.

7. A process for preparing a compound according to claim 1 which comprises the step of reacting a compound of formula:

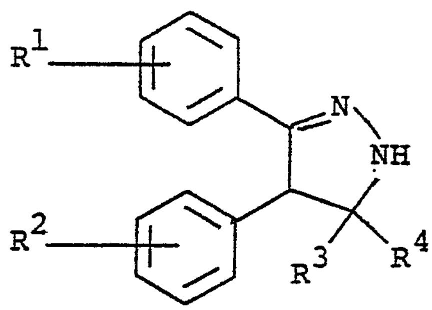

where $R^1$, $R^2$, $R^3$ and $R^4$ are as defined in claim 1 with 4-chlorophenyl isocyanate.

8. A compound of formula:

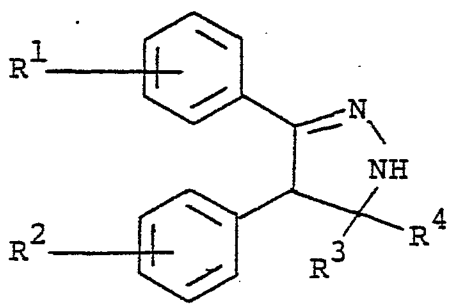

wherein $R^1$, $R^2$, $R^3$ and $R^4$ are as defined in claim 1.

9. A process for preparing a compound according to claim 8 which comprises the step of reacting a compound of formula:

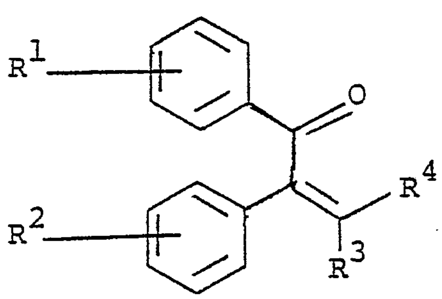

with hydrazine hydrate or an acid addition salt thereof.

10. A compound of formula:

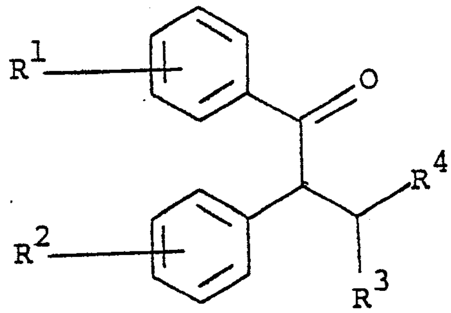

wherein $R^1$, $R^2$, $R^3$ and $R^4$ are as defined in claim 1.

11. A compound of formula:

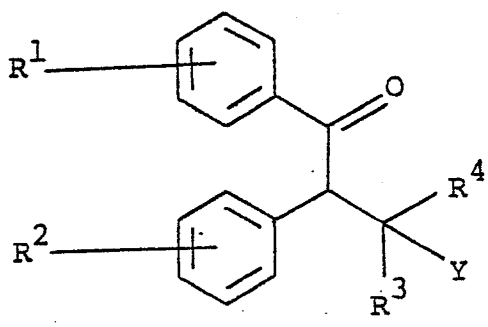

wherein $R^1$, $R^2$, $R^3$ and $R^4$ are as defined in claim 1 and Y is halo.